# EUROPEAN PATENT APPLICATION

(11) **EP 2 039 368 A2**
(43) Date of publication of application: **25.03.2009**
(21) Application number: 08021371.3
(22) Date of filing: 28.04.2000
(51) Int. Cl.: A61K 39/395, C07K 16/28, A61K 38/17, A61P 31/22

(54) **Methods for preventing reactivation of latent virus and controlling virus replication**

(30) Priority: 30.04.1999 US 131730 P
(62) Divisional of application: 00928658.4
(71) Applicant: LA JOLLA INSTITUTE FOR ALLERGY AND IMMUNOLOGY, San Diego, CA 92121 (US)
(72) Inventor: The designation of the inventor has not yet been filed
(74) Representative: Vossius & Partner

(57) **Abstract**

The invention relates to the use of a composition capable of binding to a cell membrane-expressed CD40, wherein the binding of the composition to the CD40 on the surface of the cell generates a stimulatory signal to the cell for the preparation of a pharmaceutical composition for preventing latend *Alphaherpesvirinae* virus or *Betaherpesvirinae* virus reactivation or controlling latent *Alphaherpesvirinae* virus or *Betaherpesvirinae* virus replication, wherein the composition comprises: (a) an agonist antibody that specifically binds to a cell-expressed CD40, or a composition comprising a subsequence of an anti-CD40 agonist antibody, wherein the subsequence comprises an antigen binding site that specifically binds to a cell surface CD40; or (b) a soluble CD40-ligand polypeptide that specifically binds to a cell-expressed CD40, or a composition comprising a subsequence of a CD40-ligand polypeptide, wherein the subsequence comprises a CD40 binding site that specifically binds to a cell surface CD40.

## Description

### RELATED APPLICATIONS

This application incorporates by reference and claims the benefit of priority under 35 U.S.C. § 119(e) of U.S. Provisional Application No. 60/131,730, filed April 30, 1999. The aforementioned application is explicitly incorporated herein by reference in its entirety and for all purposes

### STATEMENT AS TO FEDERALLY SPONSORED RESEARCH

This invention was made with Government support under a grant from National Institutes of Health, RO1 A145657-01. The Government may have certain rights in the invention.

### TECHNICAL FIELD

This invention generally relates to virology and medicine. In particular, this invention provides methods for preventing latent virus reactivation or controlling virus replication in an individual. The invention relates to using CD40 ligands, such as anti-CD40 antibodies and soluble CD40-ligand, as substitutes for CD4⁺ T cells in infectious disease, and more particularly to the use of CD40 ligands (soluble CD40-ligand and anti-CD40 antibodies) to prevent or inhibit viral reactivation or control virus replication.

### BACKGROUND

Chronic or persistent infections pose a serious threat to certain individuals, especially among individuals who lack a fully functional immune system, such as those that lack a T-helper cell compartment. In the absence of a functional T-helper compartment, both cell-mediated and humoral immunity are compromised. Individuals can be immunocompromised because of genetic defects, illness or disease, as a side effect of treatments, e.g., in cancer chemotherapy, or as an intentional result of therapy, as in immunosuppression to prevent graft rejections after organ or tissue transplants.

Reactivation of latent viruses can occur under these immunosuppressed or immunocompromised conditions. Viral reactivation can result in a variety of serious diseases in these patient populations. Current antiviral drugs (such as acyclovir and forscarnet) target single enzymes required for viral replication. Viral resistance to these drugs is common and toxicity is high.

CD40 and CD40L (CD154) are members of the TNF (tumor necrosis factor) superfamily of ligands and receptors. Members of this superfamily play multiple roles in innate and acquired immunity and several members of this family have costimulatory activity. CD40 is present on antigen presenting cells, including, e.g., B cells, monocytes, macrophages and dendritic cells. CD40L is present on activated CD4 T cells. CD40-CD40L interactions are known to play a role in B cell proliferation, Ig class switching, survival and memory.

### SUMMARY

The present invention includes a novel immunotherapeutic approach to the treatment of persistent and recurrent infections, particularly viral infections, using antibodies or like reagents which stimulate the CD40 molecule. This approach has significant clinical value against human viruses which cause chronic infections in immunocompromised and immunodeficient patient populations, such as those infected with human immunodeficiency virus (HIV). Viruses that can cause chronic infections in immunocompromised and immunodeficient individuals include, e.g., herpesviruses, such as cytomegalovirus (HHV-5). Varicella Zoster (HHV-3), Epstein Barr Virus (HHV-4), Herpes Simplex Virus 1 (HHV-1) and 2 (HHV-2), Karposis Sarcoma Associated Herpes Virus (HHV-8).

The invention is directed to methods for preventing latent virus reactivation or controlling virus replication in an individual comprising the following steps: (a) providing a composition capable of binding to a cell membrane-expressed CD40, wherein the binding of the composition to the CD40 on the surface of the cell generates a stimulatory signal to the cell; and (b) administering to the individual an amount of the composition sufficient to stimulate a CD40-expressing cell, thereby preventing latent virus reactivation or controlling virus replication in the individual.

In one embodiment of this method, the composition can comprise an antibody that specifically binds to a cell-expressed CD40, or a composition comprising a subsequence of an anti-CD40 antibody, wherein the subsequence comprises an antigen binding site that specifically binds to a cell surface CD40. In another embodiment, the composition can comprise a soluble CD40-ligand polypeptide that specifically binds to a cell-expressed CD40, or a composition comprising a subsequence of a CD40-ligand polypeptide, wherein the subsequence comprises a CD40 binding site that specifically binds to a cell surface CD40. In a further embodiment, the composition comprises a synthetic small molecule, e.g., an organic molecule, or a molecule from a combinatorial library, that specifically binds to a cell surface CD40.

The methods of the invention comprise stimulating any CD40 expressing cell. In alternative embodiments, the stimulated cell expressing the CD40 is a cell of the immune system, and, an epithelial cell. The immune cell can be, e.g., a B lymphocyte, a T lymphocyte, an antigen presenting cell, e.g., a macrophage, a dendritic cell, a monocyte.

In the methods of the invention, the individual can be a mammal, e.g., a human. The individual can be known to have been infected with a virus, or, the individual can be suspected of having been infected with a virus. The human, or patient, can be immunocompromised, e.g., as an individual infected with an human immunodeficiency virus, such as HIV-1, or the like. In another embodiment, the individual is immunosuppressed, e.g., because of infections, illness, as a side effect of treatments, such as radiation or cancer chemotherapy, or an intention effect of treatment, as the immunosuppression induced ancillary to tissue or organ transplantation.

In the methods of the invention, the composition can be formulated in any acceptable manner for administration to an individual, e.g., as a pharmaceutical composition further comprising a pharmaceutically acceptable excipient.

In the methods of the invention, the composition is administered to prevent latent reactivation or to control replication of any latent virus. In one embodiment, the virus is a *Herpesviridae* virus, such as an *Alphaherpesvirinae* virus, a *Betaherpesvirinae* virus, or a *Gammaherpesvirinae* virus. The *Alphaherpesvirinae* virus can be a human herpesvirus 1, a human herpesvirus 2, or a human herpesvirus 3 varicella zoster. The *Betaherpesvirinae* virus can be a human herpesvirus 5 cytomegalovirus or a human herpesvirus 6 roseolovirus. The *Gammaherpesvirinae* virus can be a *Lymphocryptovirus*. The *Lymphocryptovirus* can be a human herpesvirus 4 Epstein Barr virus (EBV). The *Herpesviridae* virus can also be a human herpesvirus 8 Kaposi's Sarcoma-associated herpesvirus or a *Herpesvirus saimiri* (HVS).

The invention further comprises a kit comprising a pharmaceutical composition comprising an antibody that specifically binds to a cell-expressed CD40, or a composition comprising a subsequence of an anti-CD40 antibody, wherein the subsequence comprises an antigen binding site that specifically binds to a cell surface CD40, And a pharmaceutically acceptable excipient, wherein the antibody or composition binds to CD40 on the surface of the cell and generates a stimulatory signal to the cell; and, printed matter comprising instructions for using the pharmaceutical composition, wherein the instructions indicate use of the pharmaceutical composition to prevent latent virus reactivation or to control virus replication.

The invention further comprises a kit comprising a pharmaceutical composition comprising a soluble CD40-ligand polypeptide that specifically binds to a cell-expressed CD40, or a composition comprising a subsequence of a CD40-ligand polypeptide, wherein the subsequence comprises a CD40 binding site that specifically binds to a cell surface CD40, and a pharmaceutically acceptable excipient, wherein the polypeptide or composition binds to CD40 on the surface of the cell and generates a stimulatory signal to the cell; and, printed matter comprising instructions for using the pharmaceutical composition, wherein the instructions indicate use of the pharmaceutical composition to prevent latent virus reactivation or to control virus replication.

The invention further comprises a method for preventing latent virus reactivation or controlling virus replication in an individual by *ex vivo* treatment and administration of antigen presenting cells, comprising the following steps: (a) providing a composition capable of binding to a cell membrane-expressed CD40, wherein the binding of the composition to the CD40 on the surface of the cell generates a stimulatory signal to the cell; (b) providing a CD40-expressing antigen presenting cell; (c) contacting the antigen presenting cell of step (b) with the composition of step (a) such that the antigen presenting cell is stimulated; (b) administering to the individual an amount of stimulated CD40-expressing antigen presenting cells sufficient to prevent latent virus reactivation or control virus replication in the individual.

In one embodiment, the composition comprises an antibody that specifically binds to a cell-expressed CD40, or a composition comprising a subsequence of an anti-CD40 antibody, wherein the subsequence comprises an antigen binding site that specifically binds to a cell surface CD40. In another embodiment, the composition comprises a soluble CD40-ligand polypeptide that specifically binds to a cell-expressed CD40, or a composition comprising a subsequence of a CD40-ligand polypeptide, wherein the subsequence comprises a CD40 binding site that specifically binds to a cell surface CD40. In a further embodiment, the composition comprises a synthetic small molecule that specifically binds to a cell surface CD40.

In one embodiment of this method, the CD40-expressing antigen presenting cell is a human cell. The CD40-expressing antigen presenting cell can be isolated from an *in vivo* source. In one embodiment, the CD40-expressing antigen presenting cell is isolated from a human. In another embodiment, the stimulated CD40-expressing antigen presenting cell is administered to the same individual from which it was isolated.

In the methods of the invention, the individual to which the stimulated cell is administered can be a mammal, e.g., a human. The individual can be known to have been infected with a virus, or, the individual can be suspected of having been infected with a virus. The human, or patient, can be immunocompromised, e.g., as an individual infected with an human immunodeficiency virus, such as HIV-1, or the like. In another embodiment, the individual is immunosuppressed, e.g., because of infections, illness, as a side effect of treatments, such as radiation or cancer chemotherapy, or an intention effect of treatment, as the immunosuppression induced ancillary to tissue or organ transplantation.

The invention also provides a method for preventing latent virus reactivation or controlling virus replication in a cell comprising the following steps: (a) providing a composition capable of binding to a cell membrane-expressed CD40, wherein the binding of the composition to the CD40 on the surface of the cell generates a stimulatory signal to the cell; and (b) contacting a viral-infected CD40-expressing cell with an amount of composition capable of stimulating the cell, thereby preventing latent virus reactivation or controlling virus replication in the cell. In alternative embodiments, the composition comprises an antibody that specifically binds to a cell-expressed CD40, or a composition comprising a subsequence of an anti-CD40 antibody, wherein the subsequence comprises an antigen binding site that specifically binds to a cell surface CD40; or, a soluble CD40-ligand polypeptide that specifically binds to a cell-expressed CD40, or a composition comprising a subsequence of a CD40-ligand polypeptide, wherein the subsequence comprises a CD40 binding site that specifically binds to a cell surface CD40; or, a synthetic small molecule that specifically binds to a cell surface CD40.

The CD40-expressing, stimulated cell can comprise any CD40 expressing cell. In alternative embodiments, the stimulated cell expressing the CD40 is a cell of the immune system, and, an epithelial cell. The immune cell can be, e.g., a B lymphocyte, a T lymphocyte, any antigen presenting cell, e.g., a macrophage, a dendritic cell, a monocyte, an activated endothelial cell, and the like. In one embodiment, the contacting is *ex vivo.* The methods of the invention can also, comprise contacting an individual with an amount of the composition capable of stimulating the CD40 expressing cell, thereby preventing latent virus reactivation or controlling virus replication in the individual.

The details of one or more embodiments of the invention are set forth in the accompanying drawings and the description below. Other features, objects, and advantages of the invention will be apparent from the description and drawings, and from the claims.

All publications, patents and patent applications cited herein are hereby expressly incorporated by reference for all purposes.

### DESCRIPTION OF DRAWINGS

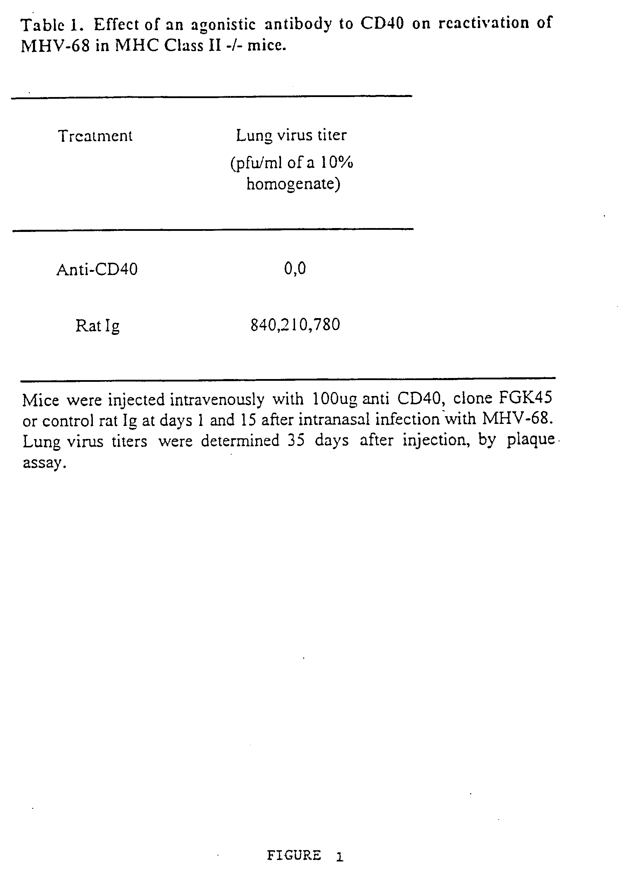

Figure 1 is Table 1, which summarizes data demonstrating the effect of an agonistic antibody to CD40 on reactivation of MHV-68 in MHC Class II -/- mice, as discussed in the Example, below.

Like reference numbers and designations in the various drawings indicate like elements.

### DETAILED DESCRIPTION

The invention provides methods for preventing latent virus reactivation or controlling virus replication in an individual or a cell. The methods involve administration of compositions that bind to cell membrane-expressed CD40 (on the surface of the cell) to generate a stimulatory signal to the cell. Stimulation, or activation, of CD40-expressing cells by this method will prevent or inhibit latent virus reactivation and will control viral replication in the individual or the cell. The methods of the invention do not have the levels of toxicity seen when conventional anti-viral drugs are administered. While the methods of the invention not limited by any mechanism of action, in one embodiment, by binding to a cell membrane-expressed CD40 and stimulating the CD40-expressing cells, the compositions administered to the individual are replacing, supplementing, or boosting responses dependent on a functional T cell compartment. This effect is particularly significant in immunocompromised and immunodeficient patient populations lacking sufficient numbers of T cells to generate a normal immune response or those individuals whose T cells are not fully functional.

Also provided is experimental data from an art-recognized animal model (a murine model) of a persistent virus infection that demonstrates that administration of a CD40 ligand, an anti-CD40 antibody, can prevent the reactivation of a latent virus, in this model, a persistent gammaherpesvirus (MHV-68). In normal mice, the MHV-68 gammaherpesvirus is controlled by the immune system and, after the initial acute infection, remains latent. However, in mice which lack a competent immune system, such as having a lack of functional T helper cells, the virus will reactivate from the latent state. In this model, the mice lack a functional T cell compartment of their immune system. Without treatment, the latent virus reactivates. This may be because of the absence of sufficient numbers of T-helper cells. As described in the Example below, mice lacking functional T helper cells were treated using the methods of the invention; particularly, with a stimulatory monoclonal antibody (FGK45) against mouse CD40; as a negative control experiment, a set of mice were treated with a control antibody (not binding to CD40). These compositions were administered at on and fifteen days after infection with MHV-68 gammaherpesvirus. All mice treated with the control antibody showed evidence of viral reactivation (release from latency) 35 days after infection. The immunodeficient mice treated with anti-CD40 showed no viral reactivation.

### DEFINITIONS

Unless defined otherwise, all technical and scientific terms used herein have the meaning commonly understood by a person skilled in the art to which this invention belongs. As used herein, the following terms have the meanings ascribed to them unless specified otherwise.

The term "amplifying" and "amplification" as used herein incorporates its common usage and refers to the use of any suitable amplification methodology for generating or detecting recombinant or naturally expressed nucleic acid, as described in detail, below. For example, reagents, *e.g*., specific degenerate oligonucleotide primer pairs, for amplifying, *e.g*., by polymerase chain reaction, PCR, naturally expressed, e.g., genomic or mRNA. or recombinant (e.g., cDNA) nucleic acids can be used to practice the methods of the invention *in vivo* or *in vitro*. For example, PCR can be used to measure the amount of viral nucleic acid or viral message in an individual before and after practicing the methods of the invention to assess the relative levels of latent, versus active, virus.

The term "antibody" refers to a peptide or polypeptide substantially encoded by an immunoglobulin gene or immunoglobulin genes, or fragments thereof, capable of specifically binding an epitope, such as, e.g., a CD40 polypeptide epitope. See, *e.g.* Fundamental Immunology, Third Edition, W.E. Paul, ed., Raven Press, N.Y. (1993); Wilson (1994) J. Immunol. Methods 175:267-73; Yarmush (1992) J. Biochem. Biophys. Methods 25:85-97. One of skill will appreciate that antibody fragments can be isolated or synthesized *de novo* either chemically or by utilizing recombinant DNA methodology. The term antibody includes antigen-binding portions (e.g., fragments, subsequences) that retain capacity to bind antigen. Examples of binding include (i) a Fab fragment, a monovalent fragment consisting of the VL, VH, CL and CH1 domains; (ii) a F(ab')2 fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward (1989) Nature 341:544-546), which consists of a VH domain; and (vi) an isolated complementarity determining region (CDR). Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules; also known as single chain Fv (scFv); see e.g., Bird (1988) Science 242:423-426; Huston (1988) Proc. Natl. Acad. Sci. USA 85:5879-5883). Single chain antibodies are also included by reference in the term "antibody." Fragments can be prepared by recombinant techniques or enzymatic or chemical cleavage of intact antibodies. The term also includes multivalent antigen-binding proteins, see, e.g., 6,027,725. The term antibody also includes "chimeric" antibodies either produced by the modification of whole antibodies or those synthesized *de novo* using recombinant DNA methodologies. Such chimeric antibodies can be "humanized antibodies," i.e., where the epitope binding site is generated from an immunized mammal, such as a mouse, and the structural framework is human. Methods for making chimeric, *e.g*., "humanized," antibodies are well known in the art, see e.g., U.S. Patent Nos. 5,811,522; 5,789,554; 6,054,297. See also, e.g., Huse (1989) Science 246:1275; Ward (1989) Nature 341:544; Hoogenboom (1997) Trends Biotechnol. 15:62-70; Katz (1997) Annu. Rev. Biophys. Biomol. Struct. 26:27-45. The term also includes human anti-human CD40 antibodies generated by transgenic non-human animals (e.g., mice comprising human Ig sequences) capable of producing entirely human antibodies, as described by, e.g., U.S. Patent Nos. 5,939,598; 5,877,397; 5,874,299; 5,814,318. The term antibody also includes epitope binding polypeptides generated using phage display libraries, and variations thereof, as described by, e.g., U.S. Patent Nos. 5,855,885; 6,027,930.

The term "CD40" or "CD40 polypeptide" means the well-characterized and described Type I cell surface receptor which is a member of the tumor necrosis factor receptor supergene family that is expressed by all antigen presenting cells, including B cells, dendritic cells, keratinocytes, monocytes, macrophages, activated endothelial cells, thymic epithelial cells. See, e.g., U.S. Patent Nos. 5,801,227; 5,874,082; 5,677,165; 6,004,552; and, 6,051,228, which describe CD40 and how to make antibodies specifically reactive with CD40, particularly antibodies reactive to human CD40.

The term "CD40 ligand" or "CD40-ligand polypeptide" means any polypeptide or peptide that specifically binds to a cell-expressed CD40, or a composition comprising a subsequence of a CD40-ligand polypeptide, wherein the subsequence comprises a CD40 binding site that specifically binds to a cell surface CD40, for example, CD154 protein. Such compositions include, e.g., "mimetic" and "peptidomimetic" and synthetic chemical compounds, as described below. See, e.g., U.S. Patent No. 5,981,724; 5,962,406; 5,817,516; 5,916,560. See also, e.g., Ford (1999) J. Immunol. 162:4037-4044; GenBank Accession Nos. P29965; AAA35662; 153476.

The terms "polypeptide," "protein," and "peptide" include compositions of the invention that also include "analogs," or "conservative variants" and "mimetics" or "peptidomimetics" with structures and activity that substantially correspond to anti-CD40 antibodies and soluble CD40 ligand, including soluble human CD40, as discussed in detail, below.

The term "pharmaceutical composition" refers to a composition suitable for pharmaceutical use in a subject. The pharmaceutical compositions of this invention are formulations that comprise a pharmacologically effective amount of a composition comprising, e.g., a CD40 ligand, or an anti-CD40 antibody, and a pharmaceutically acceptable carrier.

The term "recombinant" refers to a polynucleotide synthesized or otherwise manipulated *in vitro* (*e.g.,* "recombinant polynucleotide"), to methods of using recombinant polynucleotides to produce gene products in cells or other biological systems, or to a polypeptide ("recombinant protein") encoded by a recombinant polynucleotide. For example, recombinant CD40 binding antibodies or soluble CD40 ligand can be used to practice the methods of the invention. "Recombinant means" also encompass the ligation of nucleic acids having various coding regions or domains or promoter sequences from different sources into an expression cassette or vector for expression of, e.g., inducible or constitutive expression of polypeptide coding sequences in the vectors used to practice this invention.

### Generating and Manipulating of Nucleic Acids

The methods of the invention provide for the administration of ligands to CD40, including anti-CD40 antibodies and soluble CD40 ligand. These compositions can be administered not only as polypeptides, including recombinant polypeptides, but also in form of nucleic acids which encode CD40-binding ligands. With this mode of administration, recombinant CD40 ligands are synthesized *in vivo.* Genes encoding these compositions can be in the form of "naked DNA" or they can be incorporated in vectors for *in vivo* or *ex vivo* administration. CD40 ligands can be made and expressed *in vitro* or *in vivo*, a variety of means of making and expressing these genes and vectors can be used. One of skill will recognize that desired gene activity can be obtained by modulating the expression or activity of the genes and nucleic acids (e.g., promoters) within vectors used to practice the invention. Any of the known methods described for increasing or decreasing expression or activity, or tissue specificity, of genes can be used for this invention. The invention can be practiced in conjunction with any method or protocol known in the art, which are well described in the scientific and patent literature.

### General Techniques

The nucleic acid sequences used to practice this invention, whether RNA, cDNA, genomic DNA, vectors, viruses or hybrids thereof, may be isolated from a variety of sources, genetically engineered, amplified, and/or expressed recombinantly. Any recombinant expression system can be used, including, in addition to bacterial cells, e.g., mammalian, yeast, insect or plant cell expression systems.

Alternatively, these nucleic acids can be synthesized *in vitro* by well-known chemical synthesis techniques, as described in, e.g., Carruthers (1982) Cold Spring Harbor Symp. Quant. Biol. 47:411-418; Adams (1983) J. Am. Chem. Soc. 105:661; Belousov (1997) Nucleic Acids Res. 25:3440-3444; Frenkel (1995) Free Radic. Biol. Med. 19:373-380; Blommers (1994) Biochemistry 33:7886-7896; Narang (1979) Meth. Enzymol. 68:90; Brown (1979) Meth. Enzymol. 68:109; Beaucage (1981) Tetra. Lett. 22:1859; U.S. Patent No. 4,458,066. Double stranded DNA fragments may then be obtained either by synthesizing the complementary strand and annealing the strands together under appropriate conditions, or by adding the complementary strand using DNA polymerase with an appropriate primer sequence.

Techniques for the manipulation of nucleic acids, such as, e.g., generating mutations in sequences, subcloning, labeling probes, sequencing, hybridization and the like are well described in the scientific and patent literature, see, e.g., Sambrook, ed., MOLECULAR CLONING: A LABORATORY MANUAL (2ND ED.), Vols. 1-3, Cold Spring Harbor Laboratory, (1989); CURRENT PROTOCOLS IN MOLECULAR BIOLOGY, Ausubel, ed. John Wiley & Sons, Inc., New York (1997); LABORATORY TECHNIQUES IN BIOCHEMISTRY AND MOLECULAR BIOLOGY: HYBRIDIZATION WITH NUCLEIC ACID PROBES, Part I. Theory and Nucleic Acid Preparation, Tijssen, ed. Elsevier, N.Y. (1993).

Nucleic acids, vectors, capsids, polypeptides, and the like can be analyzed and quantified by any of a number of general means well known to those of skill in the art. These include, e.g., analytical biochemical methods such as NMR, spectrophotometry, radiography, electrophoresis, capillary electrophoresis, high performance liquid chromatography (HPLC), thin layer chromatography (TLC), and hyperdiffusion chromatography, various immunological methods, e.g. fluid or gel precipitin reactions, immunodiffusion, immuno-electrophoresis, radioimmunoassays (RIAs), enzyme-linked immunosorbent assays (ELISAs), immuno-fluorescent assays, Southern analysis, Northern analysis, dot-blot analysis, gel electrophoresis (e.g., SDS-PAGE), RT-PCR, quantitative PCR, other nucleic acid or target or signal amplification methods, radiolabeling, scintillation counting, and affinity chromatography.

### Amplification of Nucleic Acids

Oligonucleotide primers can be used to amplify nucleic acids to generate CD40 binding ligands, to monitor levels of virus and the activation state (degree of latency) of a virus, to confirm the genus and species and strains of the infecting virus, and the like. The skilled artisan can select and design suitable oligonucleotide amplification primers. Amplification methods are also well known in the art, and include, *e.g*., polymerase chain reaction, PCR (PCR PROTOCOLS, A GUIDE TO METHODS AND APPLICATIONS, ed. Innis, Academic Press, N.Y. (1990) and PCR STRATEGIES (1995), ed. Innis, Academic Press, Inc., N.Y., ligase chain reaction (LCR) (see, e.g., Wu (1989) Genomics 4:560; Landegren (1988) Science 241:1077; Barringer (1990) Gene 89:117); transcription amplification (see, e.g., Kwoh (1989) Proc. Natl. Acad. Sci. USA 86:1173); and, self-sustained sequence replication (see, e.g., Guatelli (1990) Proc. Natl. Acad. Sci. USA 87:1874); Q Beta replicase amplification (see, e.g., Smith (1997) J. Clin. Microbiol. 35:1477-1491), automated Q-beta replicase amplification assay (see, e.g., Burg (1996) Mol. Cell. Probes 10:257-271) and other RNA polymerase mediated techniques (e.g., NASBA, Cangene, Mississauga, Ontario); see also Berger (1987) Methods Enzymol. 152:307-316; Sambrook; Ausubel; U.S. Patent Nos. 4,683,195 and 4,683,202; Sooknanan (1995) Biotechnology 13:563-564.

In various embodiments of the invention, the composition is administered to prevent latent reactivation or to control replication of a *Herpesviridae* virus, such as, e.g., an *Alphaherpesvirinae* virus, a *Betaherpesvirinae* virus, or a *Gammaherpesvirinae* virus, or others, as described above. Sequences for these viruses are known in the art and can be used to design species or strain-specific diagnostic amplification primers. See, e.g., U.S. Patent No. 5,721,354.

### Cloning and construction of expression vectors

Expression vectors encoding CD40 ligands are used to express these polypeptides *in vitro* and *in vivo.* Recombinant nucleic acids expressed by a variety of conventional techniques, well described in the scientific and patent literature. See, e.g., Roberts (1987) Nature 328:731; Schneider (1995) Protein Expr. Purif. 6435:10; Sambrook, Tijssen or Ausubel. Product information from manufacturers of biological reagents and experimental equipment also provide information regarding known biological methods. The vectors can be isolated from natural sources, obtained from such sources as ATCC or GenBank libraries, or prepared by synthetic or recombinant methods.

The nucleic acids used to practice the invention can be expressed in expression cassettes, vectors or viruses which are stably or transiently expressed in cells (e.g., episomal expression systems). Selection markers can be incorporated into expression cassettes and vectors to confer a selectable phenotype on transformed cells and sequences. For example, selection markers can code for episomal maintenance and replication such that integration into the host genome is not required. For example, the marker may encode antibiotic resistance (e.g., chloramphenicol, kanamycin, G418, bleomycin, hygromycin) or herbicide resistance (e.g., chlorosulfuron or Basta) to permit selection of those cells transformed with the desired DNA sequences (see, e.g., Blondelet- Rouault (1997) Gene 190:315-317; Aubrecht (1997) J. Pharmacol. Exp. Ther. 281:992-997). Because selectable marker genes conferring resistance to substrates like neomycin or hygromycin can only be utilized in tissue culture, chemoresistance genes are also used as selectable markers *in vitro* and *in vivo.*

### Polypeptides

In various embodiments, the invention is directed to methods for preventing latent virus reactivation or controlling virus replication in a cell or an individual comprising providing a polypeptide capable of binding to a cell membrane-expressed CD40, wherein the binding of the polypeptide to the CD40 on the surface of the cell generates a stimulatory signal to the cell; and administering to the individual an amount of the polypeptide sufficient to stimulate a CD40-expressing cell, thereby preventing latent virus reactivation or controlling virus replication in the individual. The polypeptide can be a soluble CD40-ligand polypeptide that specifically binds to a cell-expressed CD40, or a composition comprising a subsequence of a CD40-ligand polypeptide, wherein the subsequence comprises a CD40 binding site that specifically binds to a cell surface CD40. The polypeptide can also be an antibody reactive with CD40, as discussed below.

As noted above, the terms "polypeptide," "protein," and "peptide," referring to the CD40 binding polypeptides used in the methods of the invention (e.g., soluble CD40 ligand and anti-CD40 antibodies) include compositions of the invention that also include "analogs," or "conservative variants" and "mimetics" or "peptidomimetics" with structures and activity that substantially correspond to anti-CD40 antibodies and soluble CD40 ligand, including soluble human CD40. Thus, the terms "conservative variant" or "analog" or "mimetic" also refer to a polypeptide or peptide which has a modified amino acid sequence, such that the change(s) do not substantially alter the polypeptide's (the conservative variant's) structure and/or activity (e.g., ability to bind to human CD40 and stimulate the bound cell). These include conservatively modified variations of an amino acid sequence, i.e., amino acid substitutions, additions or deletions of those residues that are not critical for protein activity, or substitution of amino acids with residues having similar properties (e.g., acidic, basic, positively or negatively charged, polar or non-polar, etc.) such that the substitutions of even critical amino acids does not substantially alter structure and/or activity. Conservative substitution tables providing functionally similar amino acids are well known in the art. For example, one exemplary guideline to select conservative substitutions includes (original residue followed by exemplary substitution): ala/gly or ser; arg/ lys; asn/ gln or his; asp/glu; cys/ser; gln/asn; gly/asp; gly/ala or pro; his/asn or gln; ile/leu or val; leu/ile or val; lys/arg or gln or glu; met/leu or tyr or ile; phe/met or leu or tyr; ser/thr; thr/ser; trp/tyr; tyr/trp or phe; val/ile or leu. An alternative exemplary guideline uses the following six groups, each containing amino acids that are conservative substitutions for one another: 1) Alanine (A), Serine (S), Threonine (T); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); and 6) Phenylalanine (F), Tyrosine (Y), Tryptophan (W); (see also, e.g., Creighton (1984) Proteins, W.H. Freeman and Company; Schulz and Schimer (1979) Principles of Protein Structure, Springer-Verlag). One of skill in the art will appreciate that the above-identified substitutions are not the only possible conservative substitutions. For example, for some purposes, one may regard all charged amino acids as conservative substitutions for each other whether they are positive or negative. In addition, individual substitutions, deletions or additions that alter, add or delete a single amino acid or a small percentage of amino acids in an encoded sequence can also be considered "conservatively modified variations."

The terms "mimetic" and "peptidomimetic" refer to a synthetic chemical compound that has substantially the same structural and/or functional characteristics of polypeptides used to practice the methods of the invention (e.g., CD40 ligands or anti-CD40 antibodies). The mimetic can be either entirely composed of synthetic, non-natural analogues of amino acids, or, is a chimeric molecule of partly natural peptide amino acids and partly non-natural analogs of amino acids. The mimetic can also incorporate any amount of natural amino acid conservative substitutions as long as such substitutions also do not substantially alter the mimetics' structure and/or activity. As with polypeptides of the invention which are conservative variants, routine experimentation will determine whether a mimetic is within the scope of the invention, i.e., that its structure and/or function is not substantially altered. Polypeptide mimetic compositions can contain any combination of non-natural structural components, which are typically from three structural groups: a) residue linkage groups other than the natural amide bond ("peptide bond") linkages; b) non-natural residues in place of naturally occurring amino acid residues; or c) residues which induce secondary structural mimicry, i.e., to induce or stabilize a secondary structure, e.g., a beta turn, gamma turn, beta sheet, alpha helix conformation, and the like. A polypeptide can be characterized as a mimetic when all or some of its residues are joined by chemical means other than natural peptide bonds. Individual peptidomimetic residues can be joined by peptide bonds, other chemical bonds or coupling means, such as, e.g., glutaraldehyde, N-hydroxysuccinimide esters, bifunctional maleimides, N,N'-dicyclohexylcarbodiimide (DCC) or N,N'-diisopropylcarbodiimide (DIC). Linking groups that can be an alternative to the traditional amide bond ("peptide bond") linkages include, e.g., ketomethylene (e.g., -C(=O)-CH₂- for -C(=O)-NH-), aminomethylene (CH₂-NH), ethylene, olefin (CH=CH), ether (CH₂-O), thioether (CH₂-S), tetrazole (CN₄-), thiazole, retroamide, thioamide, or ester (see, e.g., Spatola (1983) in Chemistry and Biochemistry of Amino Acids, Peptides and Proteins, Vol. 7, pp 267-357, "Peptide Backbone Modifications," Marcell Dekker, NY). A polypeptide can also be characterized as a mimetic by containing all or some non-natural residues in place of naturally occurring amino acid residues; non-natural residues are well described in the scientific and patent literature.

The structure of these polypeptides can be based on sequences of CD40, including human CD40, which are well known in the art; for example, human CD40 ligand sequence is (see, e.g., Hollenbaugh (1992) EMBO J. 11:4313-4321):

1 mietynqtsp rsaatglpis mkifmylltv flitqmigsa lfavylhrrl dkiedemlh
61 edfvfmktiq rcntgersls llnceeiksq fegfvkdiml nkeetkkens femqkgdqnp
121 qiaahvisea sskttsvlqw aekgyytmsn nlvtlengkq ltvkrqglyy iyaqvtfcsn
181 reassqapfi aslclkspgr ferillraan thssakpcgq qsihlggvfe lqpgasvfvn
241 vtdpsqvshg tgftsfgllk l

The skilled artisan will recognize that individual synthetic residues and polypeptides incorporating mimetics can be synthesized using a variety of procedures and methodologies, which are well described in the scientific and patent literature, e.g., Organic Syntheses Collective Volumes, Gilman, et al. (Eds) John Wiley & Sons, Inc., NY. Polypeptides incorporating mimetics can also be made using solid phase synthetic procedures, as described, e.g., by U.S. Pat. No. 5,422,426. Peptides and peptide mimetics of the invention can also be synthesized using combinatorial methodologies. Various techniques for generation of peptide and peptidomimetic libraries are well known, and include, *e.g*., multipin, tea bag, and split-couple-mix techniques; see, e.g., al-Obeidi (1998) Mol. Biotechnol. 9:205-223; Hruby (1997) Curr. Opin. Chem. Biol. 1:114-119; Ostergaard (1997) Mol. Divers. 3:17-27; Ostresh (1996) Methods Enzymol. 267:220-234. Modified polypeptide and peptides can be further produced by chemical modification methods, see, e.g., Belousov (1997) Nucleic Acids Res. 25:3440-3444; Frenkel (1995) Free Radic. Biol. Med. 19:373-380; Blommers (1994) Biochemistry 33:7886-7896.

These peptides can also be synthesized, whole or in part, using chemical methods well known in the art (see e.g., Caruthers (1980) Nucleic Acids Res. Symp. Ser. 215-223; Horn (1980) Nucleic Acids Res. Symp. Ser. 225-232; Banga, A.K., Therapeutic Peptides and Proteins, Formulation, Processing and Delivery Systems (1995) Technomic Publishing Co., Lancaster, PA ("Banga")). For example, peptide synthesis can be performed using various solid-phase techniques (see e.g., Roberge (1995) Science 269:202; Merrifield (1997) Methods Enzymol. 289:3-13) and automated synthesis may be achieved, e.g., using the ABI 431 A Peptide Synthesizer (Perkin Elmer) in accordance with the instructions provided by the manufacturer.

In the methods of the invention, the binding of the CD40 ligand to the CD40 on the surface of the cell generates a stimulatory signal to the cell. Methods and objective tests for determining whether, and to what extent, a CD40 ligand or a composition comprising a subsequence of a CD40-ligand polypeptide generates a stimulatory signal to a cell are well known in the art and include, e.g., measuring proliferation, cytokine secretion, expression of cell surface molecules, and other measurable phenotypic changes.

In various embodiments, this cell stimulation results in the prevention of latent virus reactivation or the controlling of virus replication in the individual. Methods and objective tests for determining whether, and to what extent, a CD40 ligand or a composition comprising a subsequence of a CD40-ligand polypeptide prevent latent virus reactivation are well known in the art and include, e.g., measuring viral titers or virus message, or, animal models, as described in the Example below. The frequency of latently-infected lymphocytes can be determined using an infectious centers assay; titers of replicating virus can be determined by plaque assay; as described in the Example, below.

### Antibodies and Immunogenic Peptides and Polypeptides

In various embodiment, antibodies to CD40 polypeptides are used to practice the methods of the invention. A variety of anti-CD40 antibodies are known in the art, e.g., see U.S. Patent Nos. 6,051,228; 6,004,552; 5,962,406; 5,817,516; 5,916,560; 5,874,082; 5,801,227; 5,677,165; 5,540,926, which describe CD40 and how to make antibodies specifically reactive with CD40, particularly antibodies reactive to human CD40.

Alternatively, using routine methods, one skilled in the art can generate antibodies to CD40. Methods of producing polyclonal and monoclonal antibodies are known to those of skill in the art and described in the scientific and patent literature, see, *e.g*., Coligan, CURRENT PROTOCOLS IN IMMUNOLOGY, Wiley/Greene, NY (1991); Stites (eds.) BASIC AND CLINICAL IMMUNOLOGY (7th ed.) Lange Medical Publications, Los Altos, CA ("Stites"); Goding, MONOCLONAL ANTIBODIES: PRINCIPLES AND PRACTICE (2d ed.) Academic Press, New York, NY (1986); Kohler (1975) Nature 256:495; Harlow (1988) ANTIBODIES, A LABORATORY MANUAL, Cold Spring Harbor Publications, New York.

Immunogenic peptides capable of generating an immune response, particularly antibodies, specifically directed to CD40, can be designed based on known CD40 polypeptide sequences, e.g., the human CD40 polypeptide sequence is (see, e.g., Stamenkovic (1989) EMBO J. 8:1403-1410; GenBank Accesssion No. 001241):

1 mvrlplqcvl wgclltavhp epptacrekq ylinsqccsl cqpgqklvsd cteftetecl

61 pcgesefldt wnrethchqh kycdpnlglr vqqkgtsetd tictceegwh ctseacescv

121 lhrscspgfg vkqiatgvsd ticepcpvgf fsnvssafek chpwtscetk dlvvqqagtn

181 ktdvvcgpqd rlralvvipi ifgilfaill vlvfikkvak kptnkaphpk qepqeinfpd

241 dlpgsntaap vqetlhgcqp vtqedgkesr isvqerq

These peptides can be isolated from natural sources (e.g., as fragments of isolated CD40 polypeptides), or can be synthetically or recombinantly generated polypeptides, as described above.

In the methods of the invention, the binding of the antibody to CD40 on the surface of the cell generates a stimulatory signal to the cell. Methods and objective tests for determining whether, and to what extent, an antibody reactive with CD40 or a composition comprising a subsequence of a CD40 antibody generates a stimulatory signal to a cell are well known in the art and include, e.g., measuring proliferation, cytokine secretion, expression of cell surface molecules, and other measurable phenotypic changes.

In various embodiments, this cell stimulation results in the prevention of latent virus reactivation or the controlling of virus replication in a cell or an individual. Methods and objective tests for determining whether, and to what extent, an anti-CD40 antibody or a composition comprising a subsequence of a CD40 antibody prevent latent virus reactivation are well known in the art and include, e.g., measuring viral titers or virus message, or, animal models, as described in the Example below. The frequency of latently-infected lymphocytes can be determined using an infectious centers assay; titers of replicating virus can be determined by plaque assay; as described in the Example, below.

### Small Molecules Reactive to CD40

In various embodiments, the composition that binds to CD40 to generate a stimulatory signal to the cell and prevent latent virus reactivation or control virus replication in a cell or individual comprises a synthetic small molecule, e.g., an organic molecule, or a molecule from a combinatorial library, that specifically binds to a cell surface CD40. Such compounds can be screened by routine methodologies, e.g., any of the assays for compounds capable of binding CD40 are amenable to high throughput screening. High throughput screening systems are commercially available (*see, e.g.,* Zymark Corp., Hopkinton, MA; Air Technical Industries, Mentor, OH; Beckman Instruments, Inc. Fullerton, CA; Precision Systems, Inc., Natick, MA, *etc*.). These systems typically automate entire procedures including all sample and reagent pipetting, liquid dispensing, timed incubations, and final readings of the microplate in detector(s) appropriate for the assay. These configurable systems provide high thru-put and rapid start up as well as a high degree of flexibility and customization. The manufacturers of such systems provide detailed protocols the various high throughput. Thus, for example, Zymark Corp. provides technical bulletins describing screening systems for detecting the modulation of gene transcription, ligand binding, and the like.

Sources for such compositions include, e.g., combinatorial chemical libraries. A combinatorial chemical library is a collection of diverse chemical compounds generated by either chemical synthesis or biological synthesis by combining a number of chemical "building blocks" such as reagents. For example, a linear combinatorial chemical library such as a polypeptide library is formed by combining a set of chemical building blocks called amino acids in every possible way for a given compound length (i.e., the number of amino acids in a polypeptide compound). Millions of chemical compounds can be synthesized through such combinatorial mixing of chemical building blocks. For example, the systematic, combinatorial mixing of 100 interchangeable chemical building blocks results in the theoretical synthesis of 100 million tetrameric compounds or 10 billion pentameric compounds (Gallop (1994) 37(9):1233-1250). Preparation and screening of combinatorial chemical libraries are well known to those of skill in the art, see, e.g., U.S. Patent Nos. 6,054,047; 6,046,056; 6,045,671; 5,792,431; 5,780,754; 5,646,046.

### Formulation and Administration of Polypeptide Pharmaceutical Compositions

In various embodiments of the invention, polypeptides, including CD40-ligand polypeptides and antibodies to CD40, are administered to an individual in amounts sufficient to prevent latent virus reactivation or to control virus replication in the individual. In further embodiments, these polypeptides are administered as pharmacological compositions; i.e., the polypeptides are formulated with a pharmaceutically acceptable carrier(s) (excipient) to form the pharmacological composition.

Pharmaceutically acceptable carriers and formulations for peptides and polypeptide are known to the skilled artisan and are described in detail in the scientific and patent literature, see e.g., the latest edition of Remington's Pharmaceutical Science, Maack Publishing Company, Easton, PA ("Remington's"); Banga; Putney (1998) Nat. Biotechnol. 16:153-157; Patton (1998) Biotechniques 16:141-143; Edwards (1997) Science 276: 1868-1871; U.S. Patent Nos. 5,780,431; 5,770,700; 5,770,201.

The polypeptide compositions used in the methods of the invention can be delivered alone or as pharmaceutical compositions by any means known in the art, *e.g*., systemically, regionally, or locally; by intraarterial, intrathecal (IT), intravenous (IV), parenteral, intra-pleural cavity, topical, oral, or local administration, as subcutaneous, intratracheal (e.g., by aerosol) or transmucosal (e.g., buccal, bladder, vaginal, uterine, rectal, nasal mucosa). Actual methods for delivering compositions will be known or apparent to those skilled in the art and are described in detail in the scientific and patent literature, see e.g., Remington's.

The pharmaceutical compositions can be administered by any protocol and in a variety of unit dosage forms depending upon the method of administration, whether other anti-virals or other drugs are being administered, and the like. Dosages for typical peptide and polypeptide pharmaceutical compositions are well known to those of skill in the art. Such dosages are typically advisorial in nature and are adjusted depending on a variety of factors, e.g., the initial responses (e.g., viral titers after initial immunization), the particular therapeutic context, patient health and tolerance. The amount of pharmaceutical composition adequate to generate the desired response is defined as a "therapeutically effective dose." The dosage schedule and amounts effective for this use, i.e., the "dosing regimen," will depend upon a variety of factors, including, e.g., the severity or degree of immunosuppression or immunodeficiency, the infecting virus and accompanying diseases or conditions, the general state of the patient's health, the patient's physical status, age, pharmaceutical formulation and concentration of pharmaceutical composition, and the like. The dosage regimen also takes into consideration pharmacokinetics, i.e., the pharmaceutical composition's rate of absorption, bioavailability, metabolism, clearance, and the like, see, *e.g*., Remington. Dosages can be determined empirically, e.g., by assessing the abatement or amelioration of symptoms, or, by objective criteria, e.g., measuring levels of viral capsids (e.g., viral titers) or viral message (by, e.g., PCR, as described above), including analysis of blood or histopathology specimens. As noted above, a single or multiple administrations can be administered depending on the dosage and frequency as required and tolerated by the patient. The pharmaceutical compositions can be administered alone or in conjunction with other therapeutic treatments.

### Ex vivo treatment and re-administration of APCs

In various embodiments of the invention, latent virus reactivation is prevented and virus replication is controlled in an individual by *ex vivo* treatment and administration of antigen presenting cells APCs. APCs are stimulated with a composition capable of binding to a cell membrane-expressed CD40 and generating a stimulatory signal to the cell. The cell can be an MHC-matched cell (a tissue-typed cell). The cell can be a tissue cultured cell or it can be an APC isolated from the individual to be treated and re-administered after *ex vivo* stimulation. Any APC can be used, as described above. Methods of isolating APCs, *ex vivo* treatment in culture, and re-administration are well known in the art, see, e.g., U.S. Patent Nos. 5,192,537; 5,665,350; 5,728,388; 5,888,705; 5,962,320; 6,017,527; 6,027,488.

### Kits

The invention provides kits that contain pharmaceutical compositions used to practice the methods of the invention. The kits can contain recombinant or synthetic CD40 binding compositions. Alternatively, the kits can also contain recombinant nucleic acids, e.g., vectors, to be administered to a patient for the in vivo generation of CD40 binding ligands. The kit can contain instructional material teaching methodologies, e.g., means to administer the compositions used to practice the invention, means to infect patients or animals with therapeutic vectors, means to monitor levels of virus and assess the relative states of viral activation and viral latency, and the like.

It is understood that the examples and embodiments described herein are for illustrative purposes only and that various modifications or changes in light thereof will be suggested to persons skilled in the art and are to be included within the spirit and purview of this application and scope of the appended claims.

### EXAMPLES

The following examples are offered to illustrate, but not to limit the claimed invention.

### Example 1: Administration of CD40 binding ligand prevents the reactivation of latent herpesvirus infection

The following example demonstrates that the methods of the invention prevent the reactivation of a latent virus infection by setting forth experimental data from an art-recognized animal model (a murine model) of a persistent virus infection that demonstrates that administration of a CD40 ligand, an anti-CD40 antibody, can prevent the reactivation of a latent virus, in this model, a persistent gammaherpesvirus (MHV-68). It is also demonstrated that, like CD4 T cells, anti-CD40 treatment assists in the control of latent virus, but does not prevent the development of latency.

Murine gammaherpesvirus-68 (MHV-68) is a naturally-occurring rodent pathogen, which is closely related to Epstein Barr virus (EBV), Kaposi's sarcoma-associated human herpesvirus 8 (HHV-8) and Herpesvirus saimiri (HVS). Structurally, the virus is more closely related to HHV-8 and HVS. Intranasal (i.n.) administration of MHV-68 results in acute productive infection of lung alveolar epithelial cells and a latent infection in B lymphocytes, lung epithelial cells and possibly some other cell types. The virus induces an inflammatory infiltrate in the lungs, enlargement of the lymph nodes and spleen and an increase in the number of activated CD8⁺ T cells in the blood. MHV-68 has also been reported to induce lymphoproliferative disease (e.g., lymphoma) in immunosuppressed mice. Thus, significant aspects of the viral pathogenesis in this animal model resemble that of EBV infection in humans.

Infectious virus is cleared from the lungs by a T cell-mediated process 10 to 13 days after initial infection (see, e.g., Ehtisham (1993) J. Virol. 67:5247-5252). In normal mice, the lungs remain clear of infectious virus thereafter. In contrast, MHC Class II -/mice, which lack functional CD4 T cells, or mice rendered deficient in the latter by antibody treatment, initially clear infectious virus from the lungs. However, these animals cannot prevent reactivation of latent infection, and infectious virus reappears in the lungs 10 to 15 days later and gradually increases in titer (see, e.g., Sarawar (1997) J. Virol. 71:3916). The precise stage at which the CD4 T cells act and their mechanism of action are currently undefined. Although CD4 T cells could be required for the generation or maintenance of CD8 T cell memory, it is known that Class II -/- mice do not produce antibody to T dependent antigens (see, e.g., Cardell (1994) Adv. Immunol. 55:423). Both humoral and cell-mediated immunity may function in the long-term control of latent MHV-68. CD4 T cells may be important for the generation and/or maintenance of anti-viral immunity.

### Materials and Methods

Mice: 129/B6 mice which were homozygous (MHC Class II -/-) for the disruption of the H-IAb gene (Grusby (1995) Annu. Rev. Immunol. 13:417-435) were obtained from Dr. Hilde Cheroutre, La Jolla Institute of Allergy and Immunology (LIAI). This strain was originally derived by Dr. Mark Grusby (Harvard, see Grusby (1995) supra). Mice were bred and housed under specific pathogen free conditions in the vivarium at LIAI. Some MHC Class II -/- and +/+ C57BL/6 mice were also purchased from Taconics (Bar Harbor, ME). The genotypes of the mice were confirmed by determining the percentage of CD4 T cells in splenocyte populations by FACS analysis. Age-matched 6 to 15 week old female MHC Class II +/+ and -/- mice were used in all experiments.

### Viral infection and sampling

MHV-68 virus (Clone G2.4) was obtained from Dr. AA Nash, Edinburgh, UK (see, e.g., Dutia (1999) J. Gen. Virol. 80:2729-2736) and stocks were grown in owl monkey kidney (OMK) cells (ATCC CRL 1556). Mice were anesthetized with Avertin (2,2,2 tribromoethanol) and infected intranasally (i.n.) with 2 x 105 plaque-forming units (pfu) of the virus (unless otherwise specified) in phosphate-buffered (PBS). Thirty-five (35) days after infection, the mice were terminally anesthetized with Avertin. The lungs were removed and homogenized in medium on ice using a Tissue Tearor™ homogenizer prior to virus titration. Single cell suspensions were prepared from the spleen, as described by Allan (1990) J. Immunol. 144:3980-3986. Cell viability was determined by Trypan Blue exclusion.

### Treatment with antibodies to CD40 in vivo

Mice were treated with 100ug FGK45, a rat monoclonal antibody to mouse CD40 (see, e.g., Shepherd (1999) J. Immunol. 163:2470-2477), or with control rat immunoglobulin. The antibodies were diluted in sterile PBS and given intravenously, one and fifteen days after infection with MHV-68.

### Virus titration and infectious centers assay

Titers of replicating virus were determined by plaque assay on NIH-3T3 cells (ATCC CRL1658) as described by Cardin (1996) J. Exp. Med. 84:863-871. Briefly, dilutions of stock virus or sonicated mouse tissues were adsorbed onto NIH-3T3 monolayers for 1hr at 37°C and overlaid with carboxymethyl cellulose (CMC). After 6 days, the CMC overlay was removed, the monolayers fixed with methanol and stained with Giemsa to facilitate determination of the number of plaques. The detection limit of this assay is 10pfu/ml of a 10% tissue homogenate based on plaques recovered from homogenates of uninfected tissues spiked with known amounts of virus.

The frequency of latently-infected lymphocytes was determined using an infectious centers assay. Leukocyte suspensions prepared from lymph nodes or spleen were plated at various cell densities on monolayers of NIH-3T3 cells, incubated overnight and then overlaid with CMC. The cells were co-cultured for 5-6 days after which the overlay was removed and the number of plaques was determined as described above.

### Results: Lung Virus titers

Lung virus titers were determined 35 days after infection with MHV- 68. As expected, all three mice treated with control rat immunoglobulin showed significant viral reactivation at this timepoint (Figure 1/Table 1). However, two mice treated with agonistic antibodies to CD40 showed no viral reactivation at this time. A third anti-CD40 treated mouse died. Initially, it appeared that there might be some toxicity associated with the treatment. However, further studies on anti-CD40 treated uninfected MHC class II -/- mice and untreated MHV-68-infected mice showed that the virus infection caused sporadic deaths in mice regardless of whether they were treated with anti-CD40. Thus 2/9 untreated MHC Class II -/- MHV-68 infected mice died, while 0/6 uninfected mice treated with FGK45 died. Bacterial infections secondary to the viral infection in the lungs are a likely cause of death in these immunocompromised mice. These data suggest that the anti-CD40 mAb has immunostimulatory function in this viral model. In another round of experiments, no mice died, and the anti-CD40 treatment was similarly significantly effective in preventing reactivation of latent virus.

### Infectious centers assay

Levels of latent virus were also determined using an infectious centers assay. Levels of latency were not significantly different in anti-CD40 treated MHC Class II -/- (33 ± 17 pfu/107 splenocytes) mice and those treated with rat Ig (18 ± 7 pfu/107 splenocytes). This indicates that, like CD4 T cells, anti-CD40 treatment assists in the control of latent virus, but does not prevent the development of latency.

*Discussion:* During primary cytotoxic T cell generation, CD4 T cells may stimulate antigen-presenting cells (APCs) via CD40-CD40L interactions; see, e.g., Ridge (1998) Nature 393:474-478; Schoenberger (1998) Nature 393:480-483; Bennett (1998) Nature 393:478-480. This would enable APCs to activate CD8 T cells (after the CD4 stimulation of the APC) without any need for CD4 cells being present at the time the APC activates the CD8 "killer" T cell. *In vitro* experiments suggest that viral infection of APCs can bypass the requirement for CD4 cell help (Ridge (1998) supra). This may explain the ability of CD8 T cells to clear primary viral infections in the absence of CD4 T cell help in *vivo*. However, the requirements for CD4 cell help *in vivo* appear to vary depending on the particular virus studied, and in the control of acute, persistent or recurrent infections. Although LCMV-infected CD4 deficient or CD40L-/- mice can mount strong primary cytotoxic T cell responses, T cell memory and B cell responses can be significantly impaired (see, e.g., Matloubian (1994) J. Virol. 68:8056; Whitmire (1996) J. Virol. 70:8375; Borrow (1996) J. Exp. Med. 183:2129).

These studies in the MHV-68 model suggest that CD4 T cells are not required for primary clearance of infectious virus but may be essential for long term control of persisting latent virus. The precise stage at which the CD4 T cells act and their mechanism of action are currently undefined. Both humoral and cell-mediated immunity may be functioning in the long-term control of latent MHV-68.

B cell-deficient mice clear replicating MHV-68 from the lungs with normal kinetics and do not show viral reactivation; however, depletion of both CD4 and CD8 T cells elicits viral reactivation in B-cell deficient, but not wildtype mice. This is probably due to the presence of neutralizing antibody in the latter. A low level of viral reactivation has also observed in B cell deficient mice depleted of CD8 T cells alone. Furthermore, Class II -/mice depleted of CD8 T cells showed increased viral reactivation. Thus, T and B cells play redundant roles in the long term control of MHV-68, but CD4 T cells are important for the generation and/or maintenance of both types of anti-viral immunity (humoral and cell mediated immunity). See, e.g., Usherwood (1996) J. Gen. Virol. 77:2819-2825; Stewart (1998) J. Exp. Med. 187:1941; Cardin (1996) supra.

While the invention is not limited by any mechanism of action, the present study demonstrates the ability of agonistic antibodies to CD40 to substitute for CD4 T cells in preventing reactivation of MHV-68 virus. Surprisingly, despite the diverse array of costimulatory molecules present on CD4 T cells, agonistic antibodies to CD40 were sufficient in preventing reactivation of MHV-68. Thus anti-CD40 treated MHC class II -/mice did not show viral reactivation, whereas control mice treated with rat Ig showed significant viral reactivation (Figure 1/ Table 1). The mechanism of this effect is currently undefined; anti CD40 antibodies could be stimulating both B cell and CTL responses. However based on Schoenberger (1998) supra, anti-CD40 mediated stimulation of anti-viral CD8 "killer" or "cytotoxic" T cells (i.e., "CTL") may be important.

These data show that immunostimulation via CD40 is sufficient for the generation and maintenance of immune mechanisms that prevent viral reactivation. Furthermore, this study demonstrates a novel method of immunotherapy to prevent herpesvirus reactivation. The latter presents particular problems in immunosuppressed individuals, such as AIDS patients, who have reduced numbers of CD4 T cells.

A number of embodiments of the present invention have been described. Nevertheless, it will be understood that various modifications may be made without departing from the spirit and scope of the invention. The invention is summarized by the following items:
1. A method for preventing latent virus reactivation or controlling virus replication in an individual comprising the following steps:
   (a) providing a composition capable of binding to a cell membrane-expressed CD40, wherein the binding of the composition to the CD40 on the surface of the cell generates a stimulatory signal to the cell; and
   (b) administering to the individual an amount of the composition sufficient to stimulate a CD40-expressing cell, thereby preventing latent virus reactivation or controlling virus replication in the individual.
2. The method of item 1, wherein the composition comprises an antibody that specifically binds to a cell-expressed CD40, or a composition comprising a subsequence of an anti-CD40 antibody, wherein the subsequence comprises an antigen binding site that specifically binds to a cell surface CD40.
3. The method of item 1, wherein the composition comprises a soluble CD40-ligand polypeptide that specifically binds to a cell-expressed CD40, or a composition comprising a subsequence of a CD40-ligand polypeptide, wherein the subsequence comprises a CD40 binding site that specifically binds to a cell surface CD40
4. The method of item 1, wherein the composition comprises a synthetic small molecule that specifically binds to a cell surface CD40.
5. The method of item 1, wherein the cell is a cell of the immune system.
6. The method of item 1, wherein the cell is an epithelial cell.
7. The method of item 1, wherein the individual is a mammal.
8. The method of item 7, wherein the mammal is a human.
9. The method of item 1, wherein the individual is immunocompromised.
10. The method of item 1, wherein the individual is infected with an human immunodeficiency virus.
11. The method of item 1, wherein the individual is immunosuppressed.
12. The method of item 1, wherein the composition is a pharmaceutical composition further comprising a pharmaceutically acceptable excipient.
13. The method of item 1, wherein the virus is a *Herpesviridae* virus.
14. The method of item 13, wherein the *Herpesviridae* virus is an *Alphaherpesvirinae* virus, a *Betaherpesvirinae* virus, or a *Gammaherpesvirinae* virus.
15. The method of item 13, wherein the *Alphaherpesvirinae* virus is a human herpesvirus 1, a human herpesvirus 2, or a human herpesvirus 3 varicella zoster.
16. The method of item 13, wherein the *Betaherpesvirinae* virus is a human herpesvirus 5 cytomegalovirus or a is a human herpesvirus 6 roseolovirus.
17. The method of item 13, wherein the *Gammaherpesvirinae* virus is a *Lymphocryptovirus.*
18. The method of item 17, wherein the *Lymphocryptovirus* is a human herpesvirus 4 Epstein Barr virus (EBV).
19. The method of item 13, wherein the *Herpesviridae* virus is a human herpesvirus 8 Kaposi's Sarcoma-associated herpesvirus or a *Herpesvirus saimiri* (HVS).
20. A kit comprising a pharmaceutical composition comprising an antibody that specifically binds to a cell-expressed CD40, or a composition comprising a subsequence of an anti-CD40 antibody, wherein the subsequence comprises an antigen binding site that specifically binds to a cell surface CD40, And a pharmaceutically acceptable excipient, wherein the antibody or composition binds to CD40 on the surface of the cell and generates a stimulatory signal to the cell; and, printed matter comprising instructions for using the pharmaceutical composition, wherein the instructions indicate use of the pharmaceutical composition to prevent latent virus reactivation or to control virus replication.
21. A kit comprising a pharmaceutical composition comprising a soluble CD40-ligand polypeptide that specifically binds to a cell-expressed CD40, or a composition comprising a subsequence of a CD40-ligand polypeptide, wherein the subsequence comprises a CD40 binding site that specifically binds to a cell surface CD40, and a pharmaceutically acceptable excipient, wherein the polypeptide or composition binds to CD40 on the surface of the cell and generates a stimulatory signal to the cell; and, printed matter comprising instructions for using the pharmaceutical composition, wherein the instructions indicate use of the pharmaceutical composition to prevent latent virus reactivation or to control virus replication.
22. A method for preventing latent virus reactivation or controlling virus replication in an individual by *ex vivo* treatment and administration of antigen presenting cells, comprising the following steps:
   (a) providing a composition capable of binding to a cell membrane-expressed CD40, wherein the binding of the composition to the CD40 on the surface of the cell generates a stimulatory signal to the cell;
   (b) providing a CD40-expressing antigen presenting cell;
   (c) contacting the antigen presenting cell of step (b) with the composition of step (a) such that the antigen presenting cell is stimulated;
      (b) administering to the individual an amount of stimulated CD40-expressing antigen presenting cells sufficient to prevent latent virus reactivation or control virus replication in the individual.
23. The method of item 22, wherein the composition comprises an antibody that specifically binds to a cell-expressed CD40, or a composition comprising a subsequence of an anti-CD40 antibody, wherein the subsequence comprises an antigen binding site that specifically binds to a cell surface CD40.
24. The method of item 22, wherein the composition comprises a soluble CD40-ligand polypeptide that specifically binds to a cell-expressed CD40, or a composition comprising a subsequence of a CD40-ligand polypeptide, wherein the subsequence comprises a CD40 binding site that specifically binds to a cell surface CD40
25. The method of item 22, wherein the composition comprises a synthetic small molecule that specifically binds to a cell surface CD40.
26. The method of item 22, wherein the CD40-expressing antigen presenting cell is a human cell.
27. The method of item 22, wherein the CD40-expressing antigen presenting cell is isolated from an *in vivo* source.
28. The method of item 27, wherein the CD40-expressing antigen presenting cell is isolated from a human.
29. The method of item 28, wherein the stimulated CD40-expressing antigen presenting cell is administered to the same individual from which it was isolated.
30. A method for preventing latent virus reactivation or controlling virus replication in a cell comprising the following steps:
   (a) providing a composition capable of binding to a cell membrane-expressed CD40, wherein the binding of the composition to the CD40 on the surface of the cell generates a stimulatory signal to the cell; and
   (b) contacting a viral-infected CD40-expressing cell with an amount of composition capable of stimulating the cell, thereby preventing latent virus reactivation or controlling virus replication in the cell.
31. The method of item 30, wherein the composition comprises an antibody that specifically binds to a cell-expressed CD40, or a composition comprising a subsequence of an anti-CD40 antibody, wherein the subsequence comprises an antigen binding site that specifically binds to a cell surface CD40.
32. The method of item 30, wherein the composition comprises a soluble CD40-ligand polypeptide that specifically binds to a cell-expressed CD40, or a composition comprising a subsequence of a CD40-ligand polypeptide, wherein the subsequence comprises a CD40 binding site that specifically binds to a cell surface CD40
33. The method of item 30, wherein the composition comprises a synthetic small molecule that specifically binds to a cell surface CD40.
34. The method of item 30, wherein the cell is a cell of the immune system.
35. The method of item 30, wherein the cell is an epithelial cell.
36. The method of item 30, wherein the contacting is *ex vivo*.
37. The method of item 34, wherein the cell is an antigen presenting cell.
38. The method of item 37, wherein the antigen presenting cell is a dendritic cell, a macrophage or a B lymphocyte.
39. The method of item 30, comprising contacting an individual with an amount of the composition capable of stimulating the CD40 expressing cell, thereby preventing latent virus reactivation or controlling virus replication in the individual.

## Claims

1. Use of a composition capable of binding to a cell membrane-expressed CD40, wherein the binding of the composition to the CD40 on the surface of the cell generates a stimulatory signal to the cell for the preparation of a pharmaceutical composition for preventing latend *Alphaherpesvirinae* virus or *Betaherpesvirinae* virus reactivation or controlling latent *Alphaherpesvirinae* virus or *Betaherpesvirinae* virus replication, wherein the composition comprises:
(a) an agonist antibody that specifically binds to a cell-expressed CD40, or a composition comprising a subsequence of an anti-CD40 agonist antibody, wherein the subsequence comprises an antigen binding site that specifically binds to a cell surface CD40; or
(b) a soluble CD40-ligand polypeptide that specifically binds to a cell-expressed CD40, or a composition comprising a subsequence of a CD40-ligand polypeptide, wherein the subsequence comprises a CD40 binding site that specifically binds to a cell surface CD40.

2. The use of claim 1, wherein the cell is:
(a) a cell of the immune system; or
(b) an epithelial cell.

3. The use of any of claims 1 or 2, wherein the pharmaceutical composition is to be administered to a mammal.

4. The use of claim 3, wherein the mammal is a human.

5. The use of any of claims 1 to 4, wherein the pharmaceutical composition is to be administered to an individual which is:
(a) immunocompromised; and/or
(b) infected with an human immunodeficiency virus.

6. The use of any of claims 1 to 5, wherein the pharmaceutical composition is to be administered to an individual which is immunosuppressed.

7. The use of any of claims 1 to 6, wherein the pharmaceutical composition further comprises a pharmaceutically acceptable excipient.

8. The use of claim 1, wherein the *Alphaherpesvirinae* virus is a human herpesvirus 1, a human herpesvirus 2, or a human herpesvirus 3 varicella zoster.

9. The use of claim 1, wherein the *Betaherpesvirinae* virus is a human herpesvirus 5 cytomegalovirus or is a human herpesvirus 6 roseolovirus.

10. Use of antigen presenting cells stimulated ex vivo with a composition capable of binding to a cell membrane-expressed CD40, wherein the binding of the composition to the CD40 on the surface of the cell generates a stimulatory signal to the cell for the preparation of a pharmaceutical composition for preventing latent *Alphaherpesvirinae* virus or *Betaherpesvirinae* virus reactivation or controlling latent *Alphaherpesvirinae* virus or *Betaherpesvirinae* virus replication in an individual, wherein the composition comprises:
(a) an agonist antibody that specifically binds to a cell-expressed CD40, or a composition comprising a subsequence of an anti-CD40 agonist antibody, wherein the subsequence comprises an antigen binding site that specifically binds to a cell surface CD40; or
(b) a soluble CD40-ligand polypeptide that specifically binds to a cell-expressed CD40, or a composition comprising a subsequence of a CD40-ligand polypeptide, wherein the subsequence comprises a CD40 binding site that specifically binds to a cell surface CD40.

11. The use of claim 10, wherein the CD40-expressing antigen presenting cell is a human cell.

12. The use of any of claims 10 or 11, wherein the CD40-expressing antigen presenting cell is isolated from an *in vivo* source.

13. The use of any of claims 10 to 12, wherein the CD40-expressing antigen presenting cell is isolated from a human.

14. The use of any of claims 10 to 13, wherein the stimulated CD40-expressing antigen presenting cell is to be administered to the same individual from which it was isolated.

15. Use of a CD40-expressing cell, which has been contacted with a composition capable of binding to a cell membrane-expressed CD40, wherein the binding of the composition to the CD40 on the surface of the cell generates a stimulatory signal to the cell for the preparation of a pharmaceutical composition for preventing latent *Alphaherpesvirinae* virus or *Betaherpesvirinae* virus reactivation or controlling latent *Alphaherpesvirinae* virus or *Betaherpesvirinae* virus replication, wherein the composition comprises:
(a) an agonist antibody that specifically binds to a cell-expressed CD40, or a composition comprising a subsequence of an anti-CD40 agonist antibody, wherein the subsequence comprises an antigen binding site that specifically binds to a cell surface CD40; or
(b) a soluble CD40-ligand polypeptide that specifically binds to a cell-expressed CD40, or a composition comprising a subsequence of a CD40-ligand polypeptide, wherein the subsequence comprises a CD40 binding site that specifically binds to a cell surface CD40.

16. The use of claim 15, wherein the cell is:
(a) a cell of the immune system, or
(b) an epithelial cell.

17. The use of any of claims 15 or 16, wherein said contacting is *ex vivo*.

18. The use of any of claims 15 to 17, wherein said contacting is to be effected in an individual.

19. The use of any of claims 15 to 18, wherein the cell is an antigen presenting cell.

20. The use of claim 19, wherein the antigen presenting cell is a dendritic cell, a macrophage or a B lymphocyte.
